# NEW EUROPEAN PATENT SPECIFICATION

(11) **EP 0 991 702 B2**
(45) Date of publication and mention of the opposition decision: **19.07.2017**
(45) Mention of the grant of the patent: 22.08.2001
(21) Application number: 98928177.9
(22) Date of filing: 19.06.1998
(51) Int. Cl.: C08J 7/04

(54) **A HYDROPHILIC COATING**
HYDROPHILE BESCHICHTUNG
REVETEMENT HYDROPHILE

(30) Priority: 20.06.1997 DK 73097
(43) Date of publication of application: 12.04.2000
(73) Proprietor: Coloplast A/S, 3050 Humlebaek (DK)
(72) Inventor: MADSEN, Niels, Joergen, DK-3450 Alleroed (DK)
(86) International application number: PCT/DK1998/000264
(87) International publication number: WO 1998/058989

(56) References cited:
- EP-A- 0 289 996
- EP-A1- 0 093 093
- EP-A1- 0 591 091
- WO-A-89/09246
- WO-A-94/16747
- WO-A1-94/26336
- WO-A1-96/23600
- GB-A- 1 600 963
- US-A- 0 217 771
- US-A- 4 373 009
- US-A- 4 459 317
- US-A- 4 769 013
- US-A- 5 416 131

## Description

### FIELD OF THE INVENTION

The present invention relates to a hydrophilic coating. Furthermore, the invention relates to a medical device provided with such a hydrophilic coating.

### BACKGROUND OF THE INVENTION

It is known to coat medical devices, e.g. catheters for introduction into human cavities such as blood vessels, digestive organs and the urinary system, with a hydrophilic coating, normally as a minimum applied on that part of the surface which is introduced or comes into contact with mucous membranes, etc., during introduction of the device. Whereas such coating is not particularly smooth when dry, so that the handling of the device does not become inconvenient, it becomes extremely slippery when it is swelled with water, preferably immediately before introduction into the human body and thus ensures a substantially painless introduction with a minimum of damage on tissue.

A large number of methods are known for the production of hydrophilic surface coatings.

These methods are mainly based on the fact that the substrate to be provided with a hydrophilic surface coating, in the course of one or more process stages with intermediary drying and curing, is coated with one or more (mostly two) layers, which are brought to react with one another in various ways, e.g. by polymerisation initiated by irradiation, by graft polymerisation, by the formation of interpolymeric network structures, or by direct chemical reaction. Known hydrophilic coatings and processes for the application thereof are e.g. disclosed in Danish Patent No. 159,018, published European Patent Application Nos. EP 0 389 632, EP 0 379 156, and EP 0 454 293, European Patent No. EP 0 093 093 B2, British Patent No. 1,600,963, US Patent Nos, 4,119,094, 4,373,009, 4,792,914, 5,041,100 and 5,120,816, and into PCT Publication Nos. WO 90/05162 and WO 91/19756.

According to a method disclosed in US Patent No. 5,001,009, a hydrophilic surface coating is prepared on a substrate by applying, in two stages or in one combined stage, on the substrate a reactive or an adhesive primer layer and then the actual hydrophilic surface layer which, in this case, comprises polyvinylpyrrolidone [PVP] as the active constituent. By this method, no chemical reaction takes place between the components of the two layers applied.

Where a device of said type, e.g. a catheter for intermittent catherisation, is to remain inside the body only for a short period, there may be a risk that water will be extracted from the hydrophilic surface coating and into the body fluids in the surrounding mucous membranes etc., owing to the higher osmotic potential of said body fluids. As a result of the extraction of water, the hydrophilic surface coating will have a tendency to become less slippery and to stick to surrounding tissues, and the removal of the medical device from the body may cause pain or damage the tissue. This is especially a problem when carrying out urodynamic examinations via a catheter.

European Patent No. EP 0 217 771 describes a method of forming a hydrophilic coating in order to retain the slipperiness for a longer period of time by applying a non-reactive hydrophilic polymer surface layer to a substrate, applying to the non-reactive hydrophilic surface polymer a solution comprising a solvent and above 2% (weight per volume) of an osmolality-increasing compound selected from the group consisting of mono and disaccharides, sugar alcohols, and non-toxic organic and inorganic salts, with the proviso that the osmolality-increasing compound is not a trihaiogsnide such as Kl₃ (Kl/I₂), and evaporating the solvent.

International patent publication No. WO 94/16747 discloses a hydrophilic coating with improved retention of water on a surface, especially a surface of a medical device such as a urethra catheter, prepared by applying to the surface in one or more process steps at least one solution of components that will combine to form the hydrophilic coating. During the final step, the surface is coated with an osmolality promoting agent which is dissolved or emulgated in the solution or in the last solution to be applied, forming the hydrophilic coating in order to maintain smoothness for longer periods of time. WO 94/16747 does not disclose cross-linked coatings.

WO 89/09246 discloses solid shaped structures having a surface coated with crosslinked hydrophilic polymer, the coating being durable and exhibiting a low coefficient of friction when wet by being in contact with salt solutions, low molecular alcohols such as methanol or ethanol or body fluids. It is stated that the degree of crosslinking is critical and is to be controlled by the operating conditions chosen as too much crosslinking reduces or completely eliminates the low friction surface property, and too little crosslinking negatively affects the durability of the coating. WO 89/09246 does not disclose the presence of a water soluble or osmolality-increasing compound in the coating.

Although a hydrophilic coating including an osmolality-increasing agent shows improved properties as compared with conventionally prepared hydrophilic surface coatings, there is still a need of medical devices, especially catheters with a hydrophilic coating conserving the slipperiness for a longer period of time in order to ensure that the coating has not lost its effect when e.g. a catheter is to be retracted. Such properties are not disclosed nor indicated in any of the references mentioned above.

### BRIEF DESCRIPTION OF THE INVENTION

It has surprisingly been found that a medical device or instrument having a cross-linked hydrophilic coating comprising urea shows a significant increased water retention and a significant decrease of the friction coefficient against living tissue as compared to a device or instrument having a cross-linked hydrophilic coating without urea.

Thus, the present invention relates to a hydrophilic coating comprising a covalently cross-linked hydrophilic polymer and urea and to a method for the preparation thereof. Furthermore, the invention relates to a medical device provided with such a hydrophilic coating.

### DETAILED DESCRIPTION OF THE INVENTION

In its broadest aspect, the invention relates to a hydrophilic coating comprising a cross-linked hydrophilic polymer and urea.

More specifically, the invention relates to a hydrophilic coating suitable for coating medical devices or instruments for introduction into human cavities comprising a covalently cross-linked hydrophilic polymer, wherein said coating comprises urea.

Thus, it has been found that crosslinked hydrophilic coating systems containing urea show a significant increase in water retention and a significant decrease in friction coefficient against living tissue as compared to non-cross-linked or physically cross-linked hydrophilic polymers of the same types containing osmolality increasing ingredients.

Generally, cross-linked coatings show a higher abrasion resistance as compared to the non-cross-linked or physically cross-linked hydrophilic coatings. Without restricting the invention to any specific hypothesis it is assumed that the very low friction coefficients of the coatings of the invention as compared to existing non-cross-linked or physically cross-linked hydrophilic coatings as well as the higher durablilty of the coatings of the invention is to be ascribed to the fact that the coating of the invention is covalently crosslinked and urea is readily dissolved and leached out of the coating when wetting the same before use leaving interspaces in the crosslinked hydrophilic coating comprising water physically bound in the coating. Thus, the coating shows a very low friction and the water only slowly leaves the coating which contributes to the conservation of the slipperiness of the coating.

It is especially preferred that the cross-linking is effected through polymerisation of vinylic unsaturated groups as such cross-linking is relatively simple to control and has proven to produce hydrophilic coatings having a low friction coefficient and high durability. Other preferred cross-linking may be obtained using polycarboxyl or polyhydroxyl compounds such as polyacrylic acid or polyethylene glycols and optionally isocyanates forming cross-linked polyurethanes

Cross-linking of hydrophilic coatings according to the invention may be effected by radiation and is preferably effected by activation using UV light.

In hydrophilic coatings according to the invention urea may be used in an amount of 0.1 - 200 % (w/w) on the basis of the dry hydrophilic coating giving rise to hydrophilic coatings showing even lower friction coefficient than that of known hydrophilic coatings comprising an "osmolality-increasing compound". Preferably, sodium chloride or urea is present in an amount of 1 - 80 % (w/w), more preferred in an amount from 2 to 30 % (w/w).

The cross-linked hydrophilic coating may be any cross-linked coating.

The cross-linked hydrophilic coating may e.g. be of the type disclosed in European patent application No. EP 0 289 996 A2. Such a coating is formed and appiied to a medical device or instrument in the form of a solution containing a water-soluble polymer, more particularly polyvinylpyrrolidone or a copolymer thereof, one or more radically polymerisable vinyl monomers and a photo initiator and optionally an activator such as mercaptoacetic acid and/or organic amines and the applied solution is exposed to an UV radiation for curing purposes. The urea is incorporated in the solution applied to the medical device or instrument.

The cross-linked hydrophilic coating may also comprise a cross-linked hydrophilic polymer wherein the polymer comprises a prepolymer containing reactive sites cross-linkable through polymerisation of vinylic unsaturated groups and optionally one or more saturated polymers crosslinked through vinylic groups. In this case, urea is also incorporated in a sol or solution of a hydrophilic prepolymer containing reactive sites cross-linkable through polymerisation of vinylic unsaturated groups and optionally one or more hydrophilic polymers to be coated onto the medical device and cross-linked by activation through UV-light or radiation and optionally hydrolysed and optionally neutralised.

The cross-linked hydrophilic coating may also be a hydrophilic coating comprising a cross-linked polyvinylpyrrolidone or a copolymer of N-vinylpyrrolidone and optionally a photo initiator.

A hydrophilic coating according to the invention may be used for coating the surface or a part thereof of a wide range of products in order to impart give the surface a low friction. As examples of products which may be provided with a surface having a low friction when wet are medical instruments such as catheters, endo and laryngoscopes, tubes for feeding, or drainage or endotracheal use, condoms, barrier coatings, e.g. for gloves, wound dressings, contact. lenses, implantates, extracorporeal blood conduits, membranes e.g. for dialysis, blood filters, devices for circulatory assistance, packaging for foodstuff, razor blades, fishermen's net, conduits for wiring, water pipes having a coating inside, sports articles, cosmetic additives, mould release agents, and fishing lines and nets.

Furthermore, the invention relates to a medical device or instrument suitable for introduction into human cavities and provided with a hydrophilic coating comprising a cross-linked hydrophilic polymer, said coating comprising urea.

The invention especially relates to catheters for intermittent catherisation provided with such a cross-linked hydrophilic coating.

The medical device of the invention is preferably a catheter. The components of a cross-linked hydrophilic coating may be applied onto a catheter made from PVC or polyurethane.

In accordance preferred embodiment of the invention the coatings comprise an antibacterial agent such as a silver salt, e.g. silver sulphadiazine, an acceptable iodine source such as povidone iodine (also called polyvinylpyrrolidone iodine), chlorhexidine salts such as the gluconate, acetate, hydrochloride or the like salts or quaternary antibacterial agents such as benzalkonium chloride or other antiseptics or antibiotics. Antibacterial agents reduces the risk of infection, especially when performing urodynamic examinations.

The term "urea" used herein should be understood to comprise urea as well as which has been N-substituted or N,N-disubstituted by lower alkyl.

"Lower alkyl" is used in the present context to designate straight or branched or cyclic aliphatic groups having from 1 to 6 carbon atoms, preferably from 1 to 4 carbon atoms such as methyl, ethyl, propyl, Isopropyl or n, iso or tert.butyl.

### MATERIALS AND METHODS

### Method for Determination of the Friction and Water Retension.

The Standard Test Method for Static and Kinetic Coefficient of Friction of Plastic Film and Sheeting, ASTM D 1894 - 93 was modified for testing the friction coefficient and wear on plastic tubes and catheters.

The tubes or catheters were cut in lengths of 10 cm and fixed on a stainless steel plate with two stainless steel rods as shown in ASTM D 1894 -93. The rods had diameters comparable with the inner diameter of the tubes or catheters to keep their shape even when heavy sledges were placed upon them.

The friction was determined under water or after wetting by dipping in water for 1 minute. The pulling force from the sledge was measured in Newtons.

Water retension of the coatings was determined by measuring the friction for up to 16 or 18 minutes after dipping the tubes in water for determining the time to reach dryness.

Polyvinylpyrrolidone: Plasdone® K90 Povidone USP from International Speciality Products.

Ethanol: Absolute Alcohol.

Butyrolactone: Gamma-butyrolactone from International Speciality Products.

UV catalyst: ESACURE KIP 150 from Lamberti SpA.
The invention is further explained in detail with reference to the below working examples disclosing embodiments of the invention. The embodiments are illustrative of the principles of the invention and it is clear to the skilled in the art that modifications may be made without deviating from the gist of the invention, the scope of which is set forth in the appended claims.

### EXPERIMENTAL PART

### EXAMPLE A

### Preparation of a catheter having a cross-linked hydrophilic PVP coating.

4.9 parts of PVP K 90 and 0 1 parts ESACURE KIP 150 was dissolved in 95 parts of an ethanol/gamma butyrolactone (85/15) solvent mixture and used as a primer coat. PVC-catheters were dipped in the primer solution and dried in an oven for 1 minute at 60°C before dipping in a solution of 4 parts of PVP K 90 dissolved in 96 parts of an ethanol/gamma butyrolactone (85/15) solvent mixture. After drying for 30 minutes the PVC-catheters were exposed to UV-light with a wave length between 200 and 300 nm for 5 minutes.

### EXAMPLE B

### Preparation of a catheter having a hydrophilic coating.

PVC-catheters were dipped in a primer solution of 4 parts of a medical grade thermoplastic polyurethane and 2 parts of nitrocellulose dissolved in 94 parts of THF and afterwards dried in an oven for 15 minutes at 60°C.
4.0 parts of PVP K 90 was dissolved in 96 parts of an ethanol/gamma butyrolactone (85/15) solvent mixture and coated onto the PVC-catheters and dried 1 hour in an oven at 60°C.

### EXAMPLE 1.

### Preparation of a catheter according to the invention having a cross-linked hydrophilic coating comprising urea.

PVC-catheters were dipped in the primer solution as made in Example A, after drying 1 minute at 60°C the catheters were dipped in a topcoat solution of 3.36 parts of PVP K 90 and 0.64 parts of urea dissolved in 96 parts of an ethanol/gamma butyrolactone (85/15) solvent mixture. The coating was further dried in an oven for 30 minutes at 60°C and exposed to UV-light with a wave length between 200 and 300 nm for 5 minutes.

### COMPARATIVE EXAMPLE 1

### Preparation of a catheter having a non- crosslinked hydrophilic coating comprising urea.

PVC-catheters were dipped in the PU/nitrocellulose primer solution as made in comparative Example B and dried for 15 minutes before they were dipped in the PVP-solution containing 3.36 parts of PVP K 90, 0.64 parts of urea and 96 parts of an ethanol/gamma butyrolactone (85/15) solvent mixture. The catheters were further dried 1 hour.

### EXAMPLE 2

### Preparation of a catheter having a cross-linked hydrophilic coating comprising sodium chloride.

PVC-catheters were dipped in the primer solution as made in Example A. and dried for 1 minute at 60°C. Then the catheters were dipped in the PVP-solution of 3.6 parts of PVP K 90 and 0.4 parts sodium chloride dissolved in 96 parts of an ethanol/gamma butyrclactone/water (64/20/16) solvent mixture. The catheters were further dried for 30 minutes and exposed to UV-light with a wave length range between 200 and 300 nm. for 5 minutes.

### COMPARATIVE EXAMPLE 2

### Preparation of a catheter having a non- crosslinked hydrophilic coating comprising sodium chloride.

PVC-catheters were dipped in the PU/nitrocellulose primer solution as made in the comparative Example B and dried for 5 minutes before they were dipped in the PVP-solution containing 3.6 parts of PVP K 90 and 0,4 parts sodium chloride dissolved in 96 parts of an ethanol/gamma butyrolactone/water (64/20/16) solvent mixture.

The result of determination of the friction force of the 6 hydrophilic coatings is shown in table 1. The figures are means of 3 readings.

**Table 1**

| Friction force measured on cross-linked and non cross-linked PVP coatings and cross-linked and non cross-linked PVP coatings comprising low molecular weight compounds, urea or sodium chloride. | | | | | | |
|---|---|---|---|---|---|---|
| Example | A | B | 1 | Comp 1 | 2 | Comp 2 |
| Initial reading (N) | 0.10 | 0.07 | 0.03 | 0.07 | 0.10 | 0.3 |
| After 2 min (N) | 0.11 | 0.08 | 0.03 | 0.07 | 0.10 | 0.7 |
| After 4 min (N) | dry | 0.22 | 0.03 | 0.08 | 0.11 | dry |
| After 6 min (N) | | dry | 0.03 | 0.10 | 0.3 | |
| After 8 min (N) | | | 0.04 | dry | dry | |
| After 10 min (N) | | | 0.04 | | | |
| After 12-18 min (N) | | | dry | | | |

It appears from the table that the coatings of the invention are superior with respect to retaining low friction as compared to coatings not being cross-linked and coatings not being cross-linked but comprise an osmolality increasing agent.

### EXAMPLE 3

### Preparation of a tube having a hydrophilic coating.

A dispersion of polyisocyanate was prepared from 50 parts of a PVP/hydroxyethylmethacrylate-copolymer having 10 mole % HEMA, 10 parts polyethyleneglycol having M_{w}=10,000, 30 parts of polyisocyanate (Bayhudur® VP LS 2032 from Bayer AG) and 10 parts of sodium chloride in the form of a 10% dispersion in water. Tubes of polyurethane were dipped into the dispersion and dried at 70°C for three hours in an oven. The coating showed a low friction coefficient and high abrasion resistance.

### EXAMPLE 4

### Leaching of water soluble compound from coating of the invention

A catheter having a coating of 5 microns dry thickness according to the invention prepared according to Example 1 was tested for leaching of urea in water after 30 seconds and 24 hours.
The catheter was dipped in 60 milliliters of water and the concentration of urea was determined by a spectrophotometric method after 30 seconds and 24 hours respectively. The coating swells to about 80 microns. It is assumed that all urea had been leashed from the coating after 24 hours. The concentration of urea in the liquid was 0.0348 grams per liter after 30 seconds and 0.0373 grams per liter after 24 hours. Thus, 93.3% of the urea is leached out of the coating after 30 seconds and the urea remaining in the coating corresponds to 0.06% of the inclusion water of the coating.

Based on the results it is demonstrated that the coating of the invention has a low friction coefficient although being hypotonic.

## Claims

1. A covalently cross-linked hydrophilic coating comprising a covalently cross-linked hydrophilic polymer suitable for coating medical devices or instruments for introduction into human cavities, **characterised in that** said coating comprises urea.

2. A hydrophilic coating as claimed in claim 1, **characterised in that** the hydrophilic polymer is cross-linked via vinylic groups.

3. A hydrophilic coating as claimed in claim 1 or 2, **characterised in that** the water soluble compound is present in an amount of 0.1-200% (w/w) on the basis of the hydrophilic coating.

4. A hydrophilic coating as claimed in claim 3, **characterised in that** urea is present in an amount of 2-80% (w/w).

5. A hydrophilic coating as claimed in any of claims 1-4, **characterised in that** the cross-linked coating contains an antibacterial agent.

6. A medical device or instrument suitable for introduction into human cavities and having a covalently cross-linked hydrophilic coating comprising a covalently cross-linked hydrophilic polymer suitable for coating medical devices or instruments for introduction into human cavities, **characterised in that** said coating comprises urea.

## Patentansprüche

1. Kovalent vernetzte hydrophile Beschichtung, die ein kovalent vernetztes hydrophiles Polymer enthält und zur Beschichtung von medizinischen Vorrichtungen oder Instrumenten geeignet ist, die zum Einführen in Hohlräume des menschlichen Körpers vorgesehen sind, **dadurch gekennzeichnet, daß** die Beschichtung Harnstoff enthält.

2. Hydrophile Beschichtung nach Anspruch 1, **dadurch gekennzeichnet, daß** das hydrophile Polymer über Vinylgruppen vernetzt ist.

3. Hydrophile Beschichtung nach Anspruch 1 oder 2, **dadurch gekennzeichnet, daß** die wasserlösliche Verbindung in einem Mengenanteil von 0,1 bis 200 Gew.-%, bezogen auf die hydrophile Beschichtung, vorliegt.

4. Hydrophile Beschichtung nach Anspruch 3, **dadurch gekennzeichnet, daß** Harnstoff in einem Mengenanteil von 2 bis 80 Gew.-% vorliegt.

5. Hydrophile Beschichtung nach einem der Ansprüche 1 bis 4, **dadurch gekennzeichnet, daß** die vernetzte Beschichtung ein antibakterielles Mittel enthält.

6. Medizinische Vorrichtung oder medizinisches Instrument, die zum Einführen in Hohlräume des menschlichen Körpers geeignet sind und eine kovalent vernetzte hydrophile Beschichtung aufweisen, die ein kovalent vernetztes hydrophiles Polymer enthält und zum Beschichten von medizinischen Vorrichtungen oder Instrumenten, die zum Einführen in Hohlräume des menschlichen Körpers vorgesehen sind, geeignet ist, **dadurch gekennzeichnet, daß** die Beschichtung Harnstoff enthält.

## Revendications

1. Revêtement hydrophile réticulé par covalence comprenant un polymère hydrophile réticulé par covalence, convenant pour recouvrir des dispositifs ou instruments médicaux destinés à être introduits dans les cavités humaines, **caractérisé en ce que** ledit revêtement comprend de l'urée.

2. Revêtement hydrophile selon la revendication 1, **caractérisé en ce que** le polymère hydrophile est réticulé par l'intermédiaire de groupes vinyliques.

3. Revêtement hydrophile selon la revendication 1 ou 2, **caractérisé en ce que** le composé soluble dans l'eau est présent à raison de 0,1 à 200 % (poids/poids) par rapport au revêtement hydrophile.

4. Revêtement hydrophile selon la revendication 3, **caractérisé en ce que** l'urée est présente à raison de 2 à 80 % (poids/poids).

5. Revêtement hydrophile selon l'une quelconque des revendications 1 à 4, **caractérisé en ce que** le revêtement réticulé contient un agent antibactérien.

6. Dispositif ou instrument médical convenant pour être introduit dans les cavités humaines et présentant un revêtement hydrophile réticulé par covalence comprenant un polymère hydrophile réticulé par covalence, convenant pour recouvrir des dispositifs ou instruments médicaux destinés à être introduits dans les cavités humaines, **caractérisé en ce que** ledit revêtement comprend de l'urée.
